# EUROPÄISCHE PATENTSCHRIFT

(11) **EP 0 828 404 B1**
(45) Veröffentlichungstag und Bekanntmachung des Hinweises auf die Patenterteilung: **19.01.2005**
(21) Anmeldenummer: 97115218.6
(22) Anmeldetag: 03.09.1997
(51) Int. Cl.: H04R 25/00

(54) **Hörgerät und Steuergerät zur Programmierung des Hörgerätes**
Hearing aid and control device for programming the hearing aid
Prothèse auditive et dispositif de commande pour la programmation de la prothèse

(30) Priorität: 06.09.1996 DE 29615554 U
(43) Veröffentlichungstag der Anmeldung: 11.03.1998
(73) Patentinhaber: Türk + Türk Electronic GmbH, 51469 Bergisch Gladbach (DE)
(72) Erfinder: Türk, Hans-Herbert, Jr., 51519 Odenthal (DE)
(74) Vertreter: Selting, Günther, Dipl.-Ing.

(56) Entgegenhaltungen:
- EP-A- 0 175 909
- EP-A- 0 448 764
- EP-A- 0 480 097
- CH-A- 671 131
- US-A- 4 918 736
- US-A- 5 172 346

## Beschreibung

Die Erfindung bezieht sich auf ein Hörgerät und ein Steuergerät zur Programmierung des Hörgeräts, wobei das Steuergerät eingegebene Patientendaten in patientenbezogene Anpaßparameter umsetzt oder aus dem Hörgerät ausgelesene Anpaßparameter verändert, und die Anpaßparameter an das Hörgerät sendet.

Zur individuellen Anpassung eines Hörgerätes an einen Patienten benutzt ein Hörgeräteakustiker ein Steuergerät, mit dem das Hörgerät mit verschiedenen Anpaßparametern programmiert werden kann. In das Steuergerät werden zunächst Patientendaten eingegeben, beispielsweise Daten der Hörverlustkurve des Patienten. Mit diesen Daten werden die Werte verschiedener Anpaßparameter des Hörgerätes ermittelt, wie beispielsweise die Lage der Kniepunkte von Hochpaß- oder Tiefpaßfiltern, Lautstärkebegrenzungen wie AGCO (Automatic Gain Control Output) oder AGCI (Automatic Gain Control Input) oder frequenzabhängige Verstärkungsfaktoren. Zur Übertragung der Anpaßparameter von dem Steuergerät zu dem Hörgerät wird eine vom Steuergerät ausgehende Datenleitung benutzt, die mit einem Stecker in eine entsprechende Buchse des Hörgerätes eingesteckt wird. Die Buchse nimmt im Hörgerät im Verhältnis zu seinem Gesamtvolumen viel Raum ein, der insbesondere bei In-dem-Ohr Hörgeräten äußerst knapp ist.

Es sind auch Hörgeräte bekannt, bei denen der Stecker anstelle der Batterie in das Batteriefach eingesteckt wird, das neben den Kontakten zur Spannungsversorgung weitere Kontakte zur Informationsübertragung aufweist. Auch hier sind Bauteile (Kontakte) für die Datenübertragung und die Führung und Fixierung des Steckers vorgesehen, die wertvollen Raum in dem Hörgerät einnehmen.

Für die Programmierung von Hörgeräten verschiedener Hersteller benötigt der Hörgeräteakustiker stets eine Vielzahl von Steckern und Adaptern, um die Datenleitung an das Hörgerät anschließen zu können.

Aus EP-A-0 469 174 ist eine Ultraschall-Fernbedienung für Hörgeräte bekannt, mit der der Patient, also der. Hörgerätbenutzer, Grundfunktionen, beispielsweise die Lautstärke verstellen kann. Eine Programmierung von Anpaßparametern kann mit der Fernbedienung jedoch nicht vorgenommen werden.

In EP 0448 764 A1 wird ein Hörgerät sowie ein Steuergerät zur Programmierung des Hörgerätes offenbart, wobei das Steuergerät eingegebene Patientendaten in patientenbezogene Anpassparameter umsetzt und die Anpassparameter an das Hörgerät sendet. Das Steuergerät weist eine Übertragungsvorrichtung auf, die die zu übertragenden Anpassparameter in elektrische Signale umwandelt und diese Signale durch eine Spule aussendet. Das Hörgerät verfügt ebenfalls über eine Spule, die die Signale empfängt und die empfangenen Signale in Steuerbefehle zur Einstellung der Anpassparameter umwandeln kann.

Für die drahtlose Übertragung zwischen Hörgerät und Steuergerät werden Spulen benötigt, die im Verhältnis zu den bei Hörgeräten üblichen Dimensionen relativ groß sind.

In EP 0 480 097 A1 ist ein Verfahren beschrieben, mit dem Hörgeräte-Merkmale über den im Hörgerät vorhandenen Lautsprecher durch Schallsignale zu einem Ausgabegerät gesendet werden.

In US-A 4,918,736 wird eine Fernsteuerung zum Einstellen von Parametern eines Hörgerätes offenbart, wobei die Signalübertragung über Schallwellen erfolgt. Hierzu ist in dem Hörgerät ein separater Empfänger vorgesehen.

Aufgabe der Erfindung ist es, Bauteile, die für eine Übertragung von Anpassparametern benötigt werden, einzusparen.

Diese Aufgabe wird erfindungsgemäß durch die Merkmale des Patentanspruchs 1 gelöst.

Das Steuergerät weist eine Übertragungsvorrichtung auf, die die zu übertragenden Anpaßparameter in Schallsignale umwandelt und diese Schallsignale aussendet. Die Schallsignale empfängt das Hörgerät über sein eingebautes Mikrophon und wandelt sie in Steuerbefehle zur Einstellung der Anpaßparameter um. Die Übertragung der Anpaßparameter erfolgt also drahtlos durch Schallwellen. Es entfallen Bauteile zum mechanischen und elektrischen Ankoppeln einer Datenleitung, wie beispielsweise Stecker und Buchsen. Dadurch wird wertvoller Raum in dem Hörgerät gewonnen. Durch den Wegfall der Buchse o.ä. wird auch das äußere Erscheinungsbild des Hörgerätes verbessert. Da durch die drahtlose Übertragung eine Standard-Schnittstelle geschaffen ist, entfällt auch die Anschaffung und Bevorratung von herstellerspezifischen Steckern und Adaptern durch den Hörgeräteakustiker. Zum Empfangen der Schallsignale wird das reguläre Mikrophon des Hörgerätes verwendet, so daß die Programmierung des Hörgerätes auch bei in das Ohr eingesetztem Hörgerät vorgenommen werden kann.

Das Hörgerät enthält einen Konverter, der die eingestellten Anpaßparameter in elektrische Signale mit Schallfrequenz umwandelt, welche dem Lautsprecher des Hörgerätes zugeführt werden. Ferner weist die Übertragungsvorrichtung ein Empfangsgerät zum Empfangen der die Anpaßparameter enthaltenden Schallsignale des Hörgerätes auf, um die eingestellten Anpaßparameter des Hörgerätes aus dem Hörgerät auszulesen. Dadurch können zur Verstellung bereits mit entsprechenden Anpaßparametern programmierter Hörgeräte diese Anpaßparameter drahtlos aus dem Hörgerät in das Steuergerät ausgelesen werden. Auch für den Auslesevorgang werden keine Stecker und Adapter benötigt. Der Hörgeräteakustiker kann Veränderungen bereits programmierter Anpaßparameter vornehmen, indem er die Anpaßparameter drahtlos über Schallsignale aus dem Hörgerät ausliest, sie in dem Steuergerät verändert und anschließend wieder drahtlos über Schallsignale an das Hörgerät sendet. Er benötigt dazu keine Buchsen, Stecker, Adapter und Datenleitungen. Die Schallsignalübertragung erfolgt bidirektional.

Vorzugsweise weist das Steuergerät ein Datenverarbeitungsgexät zum Umsetzen der eingegebenen Patientendaten in Anpaßparameter und als Übertragungsvorrichtung ein separates Sendegerät auf. Dadurch kann das die Schallsignale aussendende kompakte Sendegerät einfach in die Nähe des Hörgerätes gebracht werden, so daß eine sichere und zuverlässige Übertragung der Schallsignale gewährleistet ist. Das Datenverarbeitungsgerät kann beispielsweise ein Personalcomputer sein, der außerhalb einer schallgeschützten Anpaßkabine stehen kann.

In einer bevorzugten Ausgestaltung weist das Empfangsgerät der Übertragungsvorrichtung für das Auslesen eine Schallöffnung auf, in die der Lautsprecher bzw. die Lautsprecheröffnung des Hörgerätes einführbar ist. Über den Lautsprecher werden per Schall Kontrolldaten oder Anpaßparameter des Hörgerätes an das Steuergerät übertragen. Der Schallausgang des Hörgerätes wird dazu mit dem Schalleingang des Empfangsgerätes verbunden, so daß er von dem Mikrophon des Hörgerätes akustisch getrennt ist und Rückkopplungen und daraus resultierende Störungen der Schallübertragung ausgeschlossen werden. Alternativ hierzu kann während der Zeit des Sendens das Mikrophon des Hörgerätes ausgeschaltet werden.

Die Übertragungsvorrichtung sendet vorzugsweise Schallsignale im hörbaren Bereich aus. Dadurch können die einzelnen Module des Hörgerätes, wie Mikrophon, Verstärker und Lautsprecher für die drahtlose Übertragung unverändert benutzt werden. Eine spezielle Anpassung dieser Hörgerätemodule für den Empfang und das Senden von Schallsignalen ist nicht erforderlich.

Das Sendegerät kann die Übertragung mit einem Schallsignal einer einzigen diskreten Frequenz im hörbaren Frequenzbereich vornehmen. Dieser Frequenzbereich wird in dem Hörgerät durch ein Bandfilter ausgefiltert, so daß die Störsicherheit gegen Umgebungsgeräusche bei der Datenübertragung erhöht wird.

Vorzugsweise wird am Anfang einer Schallsignalübertragung eine Kennung gesendet. Die Kennung ist ein festgelegter Code, der der Übertragung der Anpaßparameter vorangestellt ist. Dadurch wird die Übertragungssicherheit erhöht.

Im folgenden wird unter Bezugnahme auf die Zeichnungen ein Ausführungsbeispiel der Erfindung näher erläutert.

Es zeigen:
- Fig. 1: zeigt ein Steuergerät, das Anpaßparameter an das Hörgerät überträgt,
- Fig. 2: zeigt das Steuergerät aus Fig. 1, das Anpaßparameter aus dem Hörgerät ausliest,
- Fig. 3: ein Blockschaltbild eines separaten Sendegerätes, und
- Fig. 4: ein Blockschaltbild des Hörgerätes.

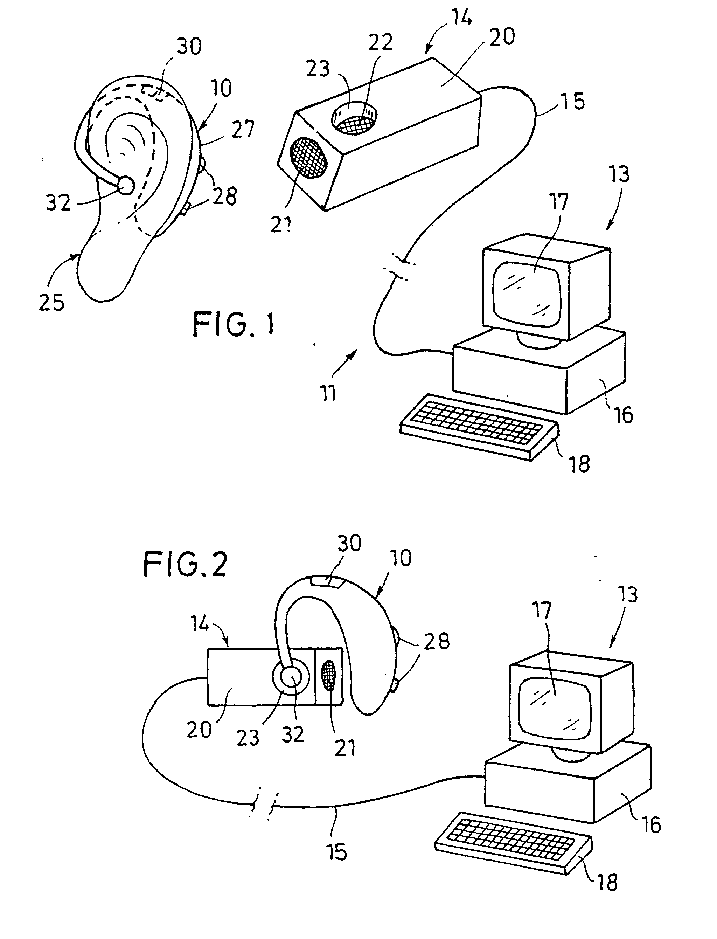

In Figur 1 ist ein Hörgerät 10 und ein Steuergerät 11 zur Programmierung eines Hinter-dem-Ohr Hörgerätes 10 dargestellt. Das Hörgerät kann jedoch auch ein In-dem-Ohr Hörgerät sein.

Das Steuergerät 11 besteht aus einem Datenverarbeitungsgerät 13, z.B. einem Personalcomputer, an den als Übertragungsvorrichtung ein separates Sende-/Empfangsgerät 14 über eine Steuerleitung 15 angeschlossen ist. Das Datenverarbeitungsgerät 13 besteht aus einer Rechnereinheit 16, einem daran angeschlossenen Monitor 17 und einer ebenfalls an die Rechnereinheit 16 angeschlossenen Tastatur 18.

Das Sende-/Empfangsgerät 14 weist ein annähernd quaderförmiges Gehäuse 20 auf, das an einer Außenfläche einen Sendelautsprecher 21 und an einer weiteren Außenfläche ein Empfängermikrophon 22 aufweist. Das Empfängermikrophon 22 ist im Bereich einer Schallöffnung 23 angeordnet. Die Schallöffnung kann auch an einem (nicht dargestellten) Stift vorgesehen sein, auf den ein mit dem Hörgerät verbundener Schlauch zur Schallübertragung aufgeschoben werden kann.

Während das Sende-/Empfangsgerät 14 in einer schallisolierten Anpaßkabine steht, ist das Datenverarbeitungsgerät 13 außerhalb dieser Kabine angeordnet. Dadurch wird eine gute, sichere und störungsfreie Übertragung der Schallsignale zwischen dem Hörgerät 10 und dem Sende-/Empfangsgerät 14 gewährleistet, ohne von den mechanischen Geräuschen des Datenverarbeitungsgerätes 13 gestört zu werden.

Das Hinter-dem-Ohr Hörgerät 10 ist auf ein Ohr 25 eines hörgeschädigten Patienten aufgesetzt. Das Hörgerät 10 hat ein bogenförmiges Gehäuse 27, dessen bauchiges Ende die Hörgerätelektronik enthält und Einstellvorrichtungen 28, wie Lautstärkeregler, Betriebsschalter etc. aufweist. Ungefähr in der Mitte des Gehäusebogens weist das Hörgerät 10 eine Mikrophon-Öffnung 30 auf. Am schlanken Ende des Hörgerätgehäuses 27 ist eine Lautsprecheröffnung 32 vorgesehen, die sich quer zur Gehäusebogenlängsachse öffnet.

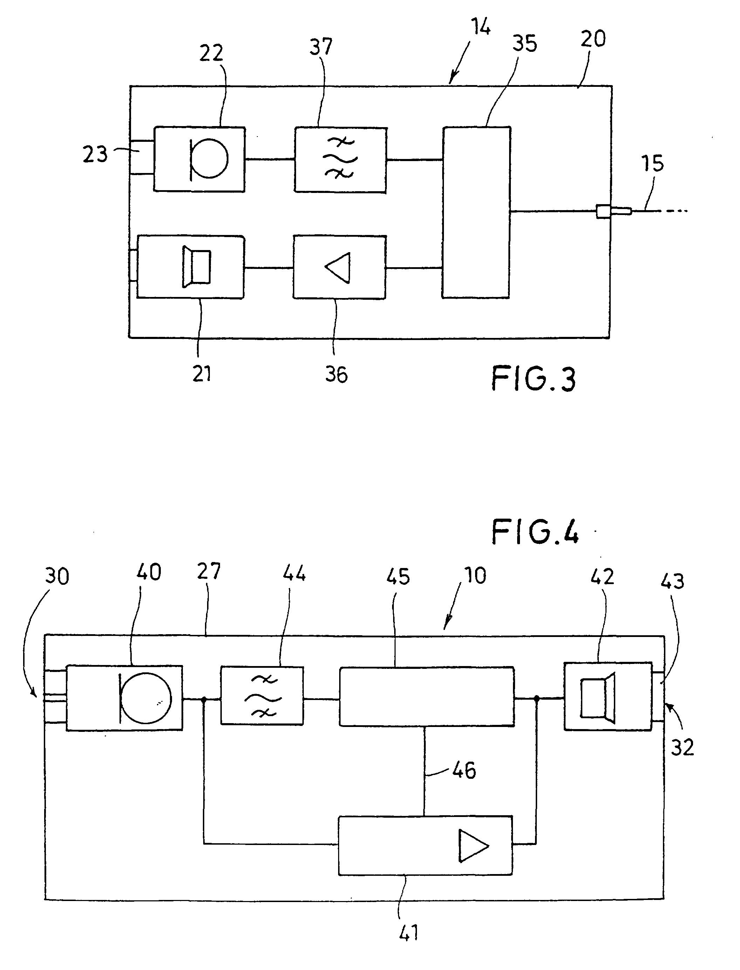

In Figur 3 ist der Aufbau des Sende-/Empfangsgerätes 14 dargestellt. Das Sende-/Empfangsgerät 14 weist ein Steuermodul 35 auf, das einerseits über die serielle Steuerleitung 15 mit der Rechnereinheit 13 verbunden ist. Andererseits weist das Steuermodul 35 eine Ausgangsleitung auf, mit der es mit einem Verstärkermodul 36 verbunden ist, das seinerseits einen Lautsprecher 21 ansteuert. Von einem Empfangsmikrophon 22 können Schallsignale empfangen werden, die dann in einem Bandfiltermodul 37 gefiltert werden, dessen Ausgang mit einem Eingang des Steuermoduls 35 verbunden ist.

In Figur 4 ist der Aufbau des Hörgerätes 10 dargestellt. Von einem Mikrophon 40, das an die Mikrophonöffnung 30 anschließt, verzweigen sich zwei Signalleitungen: Die eine Leitung führt zu einem Verstärkermodul 41, dessen Ausgang zu einem Lautsprecher 42 führt, der über eine Schalleitung 43 mit der Lautsprecheröffnung 32 verbunden ist. Die zweite Leitung führt von dem Hörgerät-Mikrophon 40 zu einem Bandfilter 44, dessen Ausgang mit einem Hörgerät-Steuermodul 45 verbunden ist. Das Hörgerät-Steuermodul 45 steuert über eine Steuerleitung 46 verschiedene Anpaßparameter des Hörgerät-Verstärkermoduls 41. Diese Anpaßparameter können Schenkelpunkte von Filterkurven, AGCO, AGCI, frequenzabhängige Verstärkungsfaktoren etc. sein. Das Hörgerät-Steuermodul 45 enthält auch einen Konverter, der die eingestellten Anpaßparameter in elektrische Signale mit Schallfrequenz umwandeln kann. Diese Signale werden dann dem Hörgerät-Lautsprecher 42 zugeführt, um Daten aus dem Hörgerät auslesen zu können. Das Steuermodul 45 ist auch mit den Einstellvorrichtungen 28 (Fig. 1,2) verbunden (nicht dargestellt).

Vor der ersten Programmierung des Hörgerätes 10, also vor der ersten Anpassung an einen neuen Patienten, werden zunächst Hördaten des hörgeschädigten Patienten in das Datenverarbeitungsgerät 13 aufgenommen. Dies kann beispielsweise dadurch geschehen, daß mit dem Datenverarbeitungsgerät 13 eine Hörverlustkurve des Patienten aufgenommen wird, die frequenzabhängig den Hörverlust darstellt. Diese Hörverlustkurve dient als Grundlage für die Ermittlung der Anpaßparameter des Hörgerätes 10. Die passenden Anpaßparameter werden beispielsweise einer in dem Datenverarbeitungsgerät 13 gespeicherten Bibliothek entnommen. Die Anpaßparameter werden in ein entsprechendes Datensignal umgewandelt, das über die Datenleitung 15. an das Sende-/Empfangsgerät 14 übertragen wird. In dem Sende-/Empfangsgerät 14 werden diese Datensignale in dem Steuermodul 35 empfangen und in entsprechende Schallsignale für die Übertragung der Anpaßparameter an das Hörgerät 10 vorbereitet. Die von dem Steuermodul 35 aufbereiteten Anpaßparameter werden dabei in kurze Impulse einer Trägerfrequenz, der Übertragungsfrequenz, umgewandelt. Diese Frequenz liegt im hörbaren Frequenzbereich. Die Signale werden von dem Sendegerät-Verstärker 36 verstärkt und zu dem Sendelautsprecher 21 übertragen, von dem die Schallsignale abgestrahlt werden. Das Hörgerät 10, das in der Nähe des Sende-/Empfangsgerätes 14 liegen kann, oder aber bereits von dem Patienten in seinem Ohr 25 getragen wird (siehe Figur 1), empfängt die von dem Sendelautsprecher 21 ausgesandten Schallsignale mit seinem Mikrophon 40.

Die von dem Hörgerät-Mikrophon 40 empfangenen Schallsignale werden sowohl zu dem Verstärkermodul 41 als auch zu dem Bandpaßfilter 44 weitergeleitet. Der Bandpaßfilter 44 ist nur für Signale der Übertragungsfrequenz durchlässig. Es werden also nur Signale der Übertragungsfrequenz an das Hörgerät-Steuermodul 45 weitergeleitet. Das Hörgerät-Steuermodul 45 dekodiert die Signale und erkennt die Übertragung von Anpaßparametern an einer der Anpaßparameterübertragung vorangestellten Kennung. Dadurch werden Übertragungsfehler und Fehleinstellungen des Hörgerätes 10 vermieden. Wenn das Hörgerät-Steuermodul 45 eine Kennung erkannt hat, werden die folgenden eingehenden Signale in entsprechende Steuersignale zur Einstellung des Verstärkermoduls 41 umgewandelt. Diese Signale werden über die Steuerleitung 46 schließlich an das Verstärkermodul 41 übertragen, so daß das Verstärkermodul 41 den Anpaßparametern entsprechend eingestellt wird. Gegebenenfalls kann das Hörgerät-Steuermodul 45 das Verstärkermodul 41 während der Einstellung des Verstärkermoduls 41 sperren, so daß die von dem Mikrophon 40 empfangenen Schallsignale nicht verstärkt und an den Lautsprecher 42 ausgegeben werden. Dadurch wird bei in das Ohr eingesetztem Hörgerät 10 eine Belästigung des Patienten durch verstärkte Steuerungs-Schallsignale vermieden.

Nach dieser ersten Grobeinstellung der Anpaßparameter folgt die Feineinstellung der Anpaßparameter. Dabei werden die Anpaßparameter solange variiert, bis eine gute und angenehme Einstellung des Hörgerätes gefunden ist.

Eine solche Feineinstellung wird nicht nur im Anschluß an eine erste Grobeinstellung, also eine Initialisierung des Hörgerätes 10 vorgenommen, sondern auch bei Hörgeräten, die bereits einmal an den Patienten angepaßt wurden, bei denen jedoch aufgrund physiologischer Veränderungen eine erneute Feinanpassung erforderlich wurde. In diesem Fall muß der Hörgeräteakustiker, insbesondere dann, wenn er über keinerlei Informationen über die Hörverlustkurve des Patienten oder die Anpaßparameter des Hörgerätes verfügt, die eingestellten Anpaßparameter aus dem Hörgerät auslesen. Dazu werden zunächst, über sein Datenverarbeitungsgerät 13 entsprechende Steuerbefehle über das Sende-/Empfangsgerät 14 an das Hörgerät-Steuermodul 45 des Hörgerätes 10 übertragen. Daraufhin veranlaßt das Steuermodul 45 einen integrierten Konverter, die eingestellten Anpaßparameter in entsprechende Signale einer bestimmten Übertragungs-Schallfrequenz zu modulieren und an den Hörgerät-Lautsprecher 42 auszugeben. Die von dem Hörgerät-Lautsprecher ausgesandten Schallsignale werden von dem Mikrophon 22 des Sende-/Empfangsgerätes 14 empfangen und anschließend in einen Bandpaßfilter 37 mit der Übertragungsfrequenz gefiltert. Das Steuermodul 35 empfängt die von dem Bandpaßfilter 37 kommenden Signale und bringt sie in vom Datenverarbeitungsgerät verarbeitbare Daten. Diese werden im Datenverarbeitungsgerät in die Anpaßparameter-Werte dekodiert, welche gespeichert werden und anschließend zur Feineinstellung des Hörgerätes 10 entsprechend verändert und an das Hörgerät 10 zurückübertragen werden können.

Durch den Einsatz der drahtlosen Datenübertragung per Schall kann bei dem Hörgerät auf Bauteile zur mechanischen Ankopplung für die Datenübertragung verzichtet werden.

## Patentansprüche

1. Hörhilfesystem mit einem Hörgerät (10) und einem Steuergerät (11) zur Programmierung des Hörgerätes (10), wobei
das Hörgerät (10) ein Mikrophon (40) und einen Lautsprecher (42) aufweist
das Steuergerät (11) derart ausgebildet ist, dass es eingegebene Patientendaten in patientenbezogene Anpassparameter umsetzt und die Anpassparameter an das Hörgerät (10) sendet,
das Steuergerät (11) eine Übertragungsvorrichtung (14) aufweist, die derart ausgebildet ist, dass sie die zu übertragenden Anpassparameter in drahtlos übermittelbare Signale umwandelt und diese Signale aussendet,
das Hörgerät (10) derart ausgebildet ist, dass es die über einen Empfänger empfangenen Signale in Steuerbefehle zur Einstellung der Anpassparameter umwandelt,
das Hörgerät (10) einen Konverter (45) enthält, der derart ausgebildet ist, dass er die eingestellten Anpassparameter in Signale umwandelt, welche einem Sender des Hörgerätes (10) zugeführt werden, und
die Übertragungsvorrichtung (14) ein Empfangsgerät (22,37,35) zum Empfangen von die Anpassparameter enthaltenden Signalen des Hörgerätes (10) aufweist,
**dadurch gekennzeichnet,**
**dass** der Hörgerät-Empfänger von dem Mikrophon (40) des Hörgerätes (10) und der Hörgerät-Sender von dem Lautsprecher (42) des Hörgerätes (10) gebildet wird,
**dass** der Konverter (45) derart ausgebildet ist, dass er die eingestellten Anpassparameter in elektrische Signale mit Schallfrequenz umwandelt,
**dass** das Steuergerät (11) derart ausgebildet ist, dass es die aus dem Hörgerät (10) ausgelesenen Anpassparameter verändert, und
**dass** die Übertragungsvorrichtung (14) derart ausgebildet ist, dass sie die Anpassparameter in Schallsignale umwandelt und diese Schallsignale aussendet.

2. Hörhilfesystem nach Anspruch 1, **dadurch gekennzeichnet, dass** das Steuergerät (11) ein Datenverarbeitungsgerät (13) zum Umsetzen der eingegebenen Patientendaten in Anpassparameter und als Übertragungsvorrichtung (14) ein separates Sendegerät (21,36,35) aufweist.

3. Hörhilfesystem nach Anspruch 1 oder 2, **dadurch gekennzeichnet, dass** die Lautsprecheröffnung (32) des Hörgerätes (10) in eine Schallöffnung (23), die mit dem Empfangsgerät (22,37,35) der Übertragungsvorrichtung (14) verbunden ist, einführbar ist.

4. Hörhilfesystem nach einem der Ansprüche 1-3, **dadurch gekennzeichnet, dass** die Übertragungsvorrichtung (14) derart ausgebildet ist, dass sie Schallsignale im hörbaren Frequenzbereich aussendet.

5. Hörhilfesystem nach Anspruch 4, **dadurch gekennzeichnet, dass** das Sendegerät (21,36,35) derart ausgebildet ist, dass es die Übertragung mit einem Schallsignal einer einzigen diskreten Frequenz im hörbaren Frequenzbereich vornimmt.

6. Hörhilfesystem nach einem der Ansprüche 1-5, **dadurch gekennzeichnet, dass** sie derart ausgebildet sind, dass am Anfang einer Schallsignal-Übertragung eine Kennung gesendet wird.

## Claims

1. A hearing aid system comprising a hearing aid (10), comprising a control device (11) for programming the hearing aid (10), wherein
the hearing aid (10) comprises a microphone (40) and a loudspeaker (42),
the control device (11) is configured to convert input patient data into patient-related tuning parameters and to transmit the tuning parameters to the hearing aid (10),
the control device (11) comprises a transmission device (14) configured to convert the tuning parameters provided for transmission into signals suited for wireless transmission, and to transmit these signals,
the hearing aid (10) is configured to convert the signals received via a receiver into control commands for setting the tuning parameters,
the hearing aid (10) includes a converter (45) configured to convert the set tuning parameters into signals which are supplied to a transmitter of the hearing aid (10), and
the transmission device (14) comprises a receiver device (22,37,35) for receiving signals from the hearing aid (10) including the tuning parameters,
**characterized in**
**that** the receiver of the hearing aid is constituted by the microphone (40) of the hearing aid (10) and the transmitter of the hearing aid is constituted by the loudspeaker (42) of the hearing aid (10),
**that** the converter (45) is configured to convert the set tuning parameters into electric signals with audio frequency,
**that** the control device (11) is configured to change the tuning parameters read from the hearing aid (10), and
**that** the transmission device (14) is configured to convert the tuning parameters into acoustic signals and to transmit these acoustic signals.

2. The hearing aid system according to claim 1, **characterized in that** the control device (11) comprises a data processing device (13) for converting the input patient data into tuning parameters, and further comprises a separate transmission unit (21,36,35) as a transmission device (14).

3. The hearing aid system according to claim 1 or 2, **characterized in that** the loudspeaker opening (32) of the hearing aid (10) is adapted for insertion into a sound aperture (23) connected to the receiver device (22,37,35) of the transmission device (14).

4. The hearing aid system according to any one of claims 1-3, **characterized in that** the transmission device (14) is configured to emit acoustic signals in the audible frequency range.

5. The hearing aid system according to claim 4, **characterized in that** the transmission unit (21,36,35) is configured to perform the transmission using an acoustic signal with a sole discrete frequency in the audible frequency range.

6. The hearing aid system according to any one of claims 1-5, **characterized by** being configured for transmission of an identification at the beginning of an acoustic signal transmission.

## Revendications

1. Système de correction auditive comprenant une prothèse auditive (10) et un appareil de commande (11) pour la programmation de la prothèse auditive (10),
la prothèse auditive (10) présentant un microphone (40) et un haut-parleur (42),
l'appareil de commande (11) étant configuré pour convertir des données de patient entrées en paramètres d'adaptation concernant le patient et envoyer les paramètres d'adaptation à la prothèse auditive (10),
l'appareil de commande (11) présentant un dispositif de transmission (14) configuré pour convertir les paramètres d'adaptation à transmettre en signaux pouvant être transmis sans fil et émettre ces signaux,
la prothèse auditive (10) étant configurée pour convertir les signaux reçus par un récepteur en ordres de commande pour le réglage des paramètres d'adaptation,
la prothèse auditive (10) contenant un convertisseur (45) configuré pour convertir les paramètres d'adaptation réglés en signaux qui sont fournis à un émetteur de la prothèse auditive (10), et
le dispositif de transmission (14) présentant un appareil récepteur (22, 37, 35) pour recevoir des signaux de la prothèse auditive contenant les paramètres d'adaptation,
**caractérisé en ce que**
le récepteur de prothèse auditive est formé par le microphone (40) de la prothèse auditive (10) et l'émetteur de prothèse auditive par le haut-parleur (42) de la prothèse auditive (10),
le convertisseur (45) est configuré pour convertir les paramètres d'adaptation réglés en signaux électriques ayant une fréquence acoustique,
l'appareil de commande (11) est configuré pour modifier les paramètres d'adaptation extraits de la prothèse auditive (10), et
le dispositif de transmission (14) est configuré pour convertir les paramètres d'adaptation en signaux acoustiques et émettre ces signaux acoustiques.

2. Système de correction auditive selon la revendication 1, **caractérisé en ce que** l'appareil de commande (11) présente un appareil de traitement de données (13) pour convertir les données de patient entrées en paramètres d'adaptation et un appareil émetteur séparé (21, 36, 35) en tant que dispositif de transmission (14).

3. Système de correction auditive selon la revendication 1 ou 2, **caractérisé en ce que** l'ouverture de haut-parleur (32) de la prothèse auditive (10) peut être introduite dans une ouverture acoustique (23) qui est reliée à l'appareil récepteur (22, 37, 35) du dispositif de transmission (14).

4. Système de correction auditive selon l'une quelconque des revendications 1-3, **caractérisé en ce que** le dispositif de transmission (14) est configuré pour émettre des signaux acoustiques sur la bande de fréquence audible.

5. Système de correction auditive selon la revendication 4, **caractérisé en ce que** l'appareil émetteur (21, 36, 35) est configuré pour effectuer la transmission avec un signal acoustique d'une seule fréquence discrète sur la bande de fréquence audible.

6. Système de correction auditive selon l'une quelconque des revendications 1-5, **caractérisé en ce qu'**il est configuré pour qu'une identification soit envoyée au début d'une transmission de signal acoustique.
